# EUROPÄISCHE PATENTSCHRIFT

(11) **EP 1 131 043 B1**
(45) Veröffentlichungstag und Bekanntmachung des Hinweises auf die Patenterteilung: **04.05.2005**
(21) Anmeldenummer: 99972526.0
(22) Anmeldetag: 10.11.1999
(51) Int. Cl.: A61K 7/13, A61K 7/135

(54) **ENTSTAUBUNG PULVERFÖRMIGER MITTEL**
DE-DUSTING POWDERY AGENTS
SOLVANTS POUR MILIEUX PULVERULENTS

(30) Priorität: 20.11.1998 DE 19853653
(43) Veröffentlichungstag der Anmeldung: 12.09.2001
(73) Patentinhaber: Hans Schwarzkopf & Henkel GmbH & Co. KG, 22763 Hamburg (DE)
(72) Erfinder: WOLFF, Wolfgang, D-22941 Bargteheide (DE); AKRAM, Mustafa, D-22457 Hamburg (DE)
(74) Vertreter: Strohe-Kamp, Geertje
(86) Internationale Anmeldenummer: PCT/EP1999/008649
(87) Internationale Veröffentlichungsnummer: WO 2000/030596

(56) Entgegenhaltungen:
- EP-A- 0 583 767
- WO-A-97/07776
- DE-A- 19 600 704
- DE-A- 19 600 705
- US-A- 4 227 880

## Beschreibung

Die Erfindung betrifft die Verwendung bestimmter Lösemittel zum Entstauben von Blondierpulvern und pulverförmigen Farbstoffmischungen, sowie entsprechende Blondierund Färbemittel.
Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.
Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung üblicherweise mit einer Wasserstoffperoxidlösung vermischt werden, werden im weiteren als "Blondiermittel" bezeichnet. Alle aufgeführten Mengenangaben beziehen sich, soweit nicht anders ausgeführt, ausschließlich auf diese Zubereitungen.
Weder die pastenförmigen noch die pulverförmigen Blondiermittel, die heute auf dem Markt sind, können als optimal angesehen werden. Während die Blondierwirkung auf dem Haar als befriedigend bezeichnet werden kann, bestehen doch noch eine Reihe von Nachteilen und Problemen sowohl bei der Herstellung als auch bei der Handhabung dieser Mittel. Bei pastenförmigen Mitteln, die aus Stabilitätsgründen hochviskos eingestellt werden, können insbesondere die Dosierung und das Mischungsverhalten in der Wasserstoffperoxidlösung noch nicht befriedigen. Ferner ist das Staubverhalten bei der Konfektionierung von Pasten ein Problem. Bei pulverförmigen Mitteln stehen das Staubverhalten, sowohl bei der Herstellung als auch bei der Anwendung, sowie ebenfalls das Mischungsverhalten bei der Anwendung im Mittelpunkt der Verbesserungsbemühungen.

Bei der Formulierung von Blondiermitteln muß ferner berücksichtigt werden, daß diese Mittel häufig Ammoniumsalze, wie z.B. Ammoniumchlorid oder Ammoniumperoxidisulfat enthalten. Basische Komponenten können während der Lagerung mit diesen Salzen unter Freisetzung von Ammoniak reagieren und somit zu einer Geruchsbelästigung führen bzw. die Lagerstabilität herabsetzen.

In der EP-B1-0 560 088 wurde beispielsweise vorgeschlagen, das Staubverhalten von Blondierpulvern durch Zugabe von Ölen oder flüssigen Wachsen zu verbessern. Dieses Vorgehen führt aber auch zu einer ganzen Reihe von Nachteilen; so sind pulverförmige Mittel nach Zugabe von Ölen oder Wachsen nur noch begrenzt rieselfähig und neigen zur Klumpenbildung. Außerdem treten bei der Verwendung von Ölen und Wachsen Probleme bei der Auspülbarkeit nach der Behandlung auf, da sich diese am Haar anlagern können. Auch aus der Perspektive der Konfektionierung der Blondiermittel ist die Anwendung von Fetten und Wachsen problematisch, da diese vor dem Zusprühen geschmolzen werden müssen. Dies erfordert einen vergrößerten technischen Aufwand, wie z.B. beheizte Leitungen.

In der deutschen Anmeldung DE-A1-196 00 216 wurde weiterhin vorgeschlagen, zur Entstaubung spezielle Ether in Mengen von 4 - 20 Gew.-% bezogen auf das gesamte Blondierpulver, einzusetzen. Aber auch diese Mittel können noch nicht alle Wünsche vollständig erfüllen.

Pulverförmige Farbpulvermischungen spielen schon seit geraumer Zeit ein wichtige Rolle. In der DE-A1-32 07 037 werden beispielsweise pulverförmige Haarfärbemittel auf Basis von Farbstoffen und mindestens einem festen Verdünnungsmittel beschrieben. Auch in neueren Patenten z.B. der DE-C1-197 13 696 wird der Einsatz pulverförmiger Mittel zum Färben und Tönen menschlicher Haare beschrieben. Ebenso wie bei den Blondierpulvern stehen das Staubverhalten, sowohl bei der Herstellung als auch bei der Anwendung, sowie weiterhin das Mischungsverhalten bei der Anwendung im Mittelpunkt der Verbesserungsbemühungen. In der DE-A1-196 00 225 wurde vorgeschlagen, pulverförmige, heterogen aufgebaute Pulverhaarfarben mit Ölen, flüssigen Wachsen oder flüssigen Derivaten dieser Verbindungen zu entstauben. Dennoch bestand weiterhin Bedarf an Mitteln zur Entstaubung pulverförmiger Mittel.

Überraschenderweise wurde nun gefunden, daß sich einige Lösemittel in hervorragender Weise zur Entstaubung derartiger Pulver eignen und daß die erhaltenen Mittel sich durch ein hervorragendes Staubverhalten, sehr guter Lagerstabilität und weitere vorteilhafte Eigenschaften auszeichnen.
Ein erster Gegenstand der Erfindung ist daher die Verwendung von 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
zum Entstauben von Blondierpulvern auf Basis mindestens einer festen Peroxoverbindung sowie mindestens eines festen Alkaliträgers und pulverförmigen Farbstoffmischungen auf Basis von mindestens einem Oxidationsfarbstoffvorprodukt enthaltend übliche kosmetische Bestandteile.
Unter Estern und Ketonen sind erfindungsgemäß gesättigte, einfach oder mehrfach ungesättigte, verzweigte oder unverzweigte, aliphatische oder cycloaliphatische Verbindungen mit 2 bis 9 C-Atomen zu verstehen, die mindestens eine, die jeweilige Stoffgruppe definierende, funktionelle Gruppe tragen.
Erfindungsgemäße Lösemittel aus der Gruppe der Ester sind Propylencarbonat, Isopropylacetat, Hexylacetat sowie Ethylglykolacetat.
Erfindungsgemäße Lösemittel aus der Gruppe der Ketone sind Cyclohexanon, Methylisobutylketon sowie Methylheptylketon.

Besonders bevorzugte Lösemittel zur Entstaubung sind ausgewählt aus einer Gruppe, gebildet von Cyclohexanon und Propylencarbonat .
In einer Ausführungsform kann es bevorzugt sein, Lösemittel einzusetzen, deren Löslichkeit in Wasser bei 20 °C mehr als 1 g/l beträgt. Lösemittel mit einer Löslichkeit von 5 g/l und mehr können besonders bevorzugt sein.
In einer weiteren Ausführungsform der vorliegenden Erfindung kann es bevorzugt sein, Lösemittel mit polaren funktionellen Gruppen zu verwenden, da diese sich durch eine größere Hydrophilie auszeichnen. Derartige Lösemittel lassen sich besser aus dem Haar auswaschen und führen nicht zu Belastungen der Haare.
Ein zweiter Gegenstand sind pulver- und pastenförmige Mittel zum Blondieren menschlicher Haare auf Basis mindestens einer festen Peroxoverbindung sowie mindestens eines festen Alkaliträgers, dadurch gekennzeichnet, dass es zur Entstaubung 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
enthält.
Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.
Die Peroxoverbindungen sind in den erfindungsgemäßen Blondiermitteln bevorzugt in Mengen von 20-80 Gew.-%, insbesondere in Mengen von 40-70 Gew.-%, enthalten.
Als weitere zwingende Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetallund Erdalkalimetallhydroxide, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.
Die erfindungsgemäßen Blondiermittel enthalten Alkalisierungsmittel bevorzugt in Mengen von 10-30 Gew.-%, insbesondere 15-25 Gew.-%.
Für die Anwendung der erfindungsgemäßen Mittel auf dem Haar werden die Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondiermittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

Ein dritter Gegenstand der vorliegenden Erfindung sind pulverförmige Mittel zum Färben menschlicher Haare, enthaltend mindestens ein Oxidationsfarbstoffvorprodukt, dadurch gekennzeichnet, dass es zur Entstaubung 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
enthält.

Als Vorprodukte für Oxidationsfarbstoffe enthalten Haarfärbemittel sogenannte Entwickler- und Kupplerkomponenten. Die Entwicklerkomponenten bilden unter dem Einfluß von Oxidationsmitteln oder Luftsauerstoff, gegebenenfalls mit Hilfe spezieller Enzyme oder Metallionen als Katalysator, untereinander oder unter Kupplung mit einer oder mehreren Kupplerkomponenten die eigentlichen Farbstoffe aus. Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.
Ganz besonders bevorzugte Entwicklerkomponenten sind p-Phenylendiamin, p-Toluylendiamin, p-Aminophenol, o-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin und 4-Hydroxy-2,5,6-triaminopyrimidin.
Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol,_ 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.
Ganz besonders bevorzugte Kupplerkomponenten sind 1-Naphthol, 1,5-, 2,7- und 1,7-Dihydroxynaphthalin, 3-Aminophenol, 5-Amino-2-methylphenol, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin und 6- Methyl-1,2,3,4-tetrahydrochinoxalin.
Die erfindungsgemäßen Haarfärbemittel enthalten sowohl die Entwicklerkomponenten als auch die Kupplerkomponenten bevorzugt in einer Menge von 0,005 bis 20 Gew.-%, jeweils bezogen auf das Färbemittel ohne die Oxidationsmittelzubereitung. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem MolVerhältnis von 1 : 0,5 bis 1 : 2 enthalten sein können.
Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Die in den erfindungsgemäßen Mitteln eingesetzten direktziehenden Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Violett-1,4 D, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel enthalten die direktziehenden Farbstoffe bevorzugt in einer Menge von 0,01 bis 20 Gew.-%, bezogen auf das gesamte Färbemittel.
Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die erfindungsgemäßen Mittel enthalten die Lösemittel zur Entstaubung in Mengen von 10 und 30 Gew.-% . Erfindungsgemäß besonders bevorzugt ist eine Entstaubung mit 10 bis 20 Gew.-% eines Lösemittels. Die Angabe in Gewichtsteilen bezieht sich auf das gesamte zu entstaubende pulverförmige Mittel.
Weiterhin hat es sich als vorteilhaft erwiesen, wenn die erfindungsgemäßen pulverförmigen Mittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.
Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.
Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C₁-C₄-Alkyl-Ethers, "verschlossen". Ein Beispiel für eine solche Verbindung ist das Handelsprodukt Dehypon®LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxy-stearat (Handelsprodukt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol + 4 EO, Nonylphenol + 9 EO, Octylphenol + 3 EO und Octylphenol + 8 EO.
Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.
Als besonders vorteilhaft haben sich erfindungsgemäße Mittel erwiesen, die nichtionogene grenzflächenaktive Substanzen in Mengen von 0,5 - 10 Gew.-% enthalten.
Weiterhin können die erfindungsgemäßen Mittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.
Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C₈-C₂₂-Carbonsäuren wie Ölsäure, Stearinsäure, Isostearinsäure und Palmitinsäure.
Diese anionischen Tenside sollten bevorzugt in fester, insbesondere Pulverform vorliegen. Ganz besonders bevorzugt sind dabei bei 25°C feste Seifen, insbesondere Natriumstearat. Diese liegen bevorzugt in Mengen von 5 bis 20 Gew.-%, insbesondere 10 bis 15 Gew.-.%, vor.
Als nichtionische Tenside eignen sich insbesondere C₈-C₂₂-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.
Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.
Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie das unter der Bezeichnung Dehyquart®F 75 in Abmischung mit Cetearylalkohol erhältliche Distearoylethylhydroxyethylammoniummethosulfät.
Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.
Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vemetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine,
- Fettalkohole und Fettsäureester,
- Fettsäurealkanolamide,
- Komplexbildner wie EDTA, NTA und Phosphonsäuren,
- Quell- und Penetrationshilfsstoffe wie Carbonate, Hydrogencarbonate, Guanidine, Harnstoffe sowie primäre, sekundäre und tertiäre Phosphate,
Die Auswahl dieser weiteren Stoffe wird der Fachmann gemäß der gewünschten Eigenschaften der Mittel treffen.
In einer bevorzugten Ausführungsform, insbesondere wenn das Haar nicht übermäßig beschwert werden soll, sind die erfindungsgemäßen Mittel im übrigen frei von Ölen und flüssigen Wachsen. Dabei ist klarzustellen, daß der Begriff Öle die bekannten fetten und synthetischen Öle, nicht aber Parfümöle umfaßt, die selbstverständlich in geringen Mengen als Duftstoffe eingesetzt werden können.
Die Herstellung der erfindungsgemäßen pulverförmigen Mittel kann nach den üblichen, dem Fachmann bekannten Verfahren erfolgen.
Ein bevorzugtes Verfahren besteht darin, die anorganischen, als Feststoff vorliegenden Komponenten nach Mischung, z.B. in einem Drais-Mischer, vorzulegen und gegebenenfalls mit dem Lösemittel zu besprühen. Dies erfolgt bevorzugt bei Raumtemperatur, d. h. bei Temperaturen unterhalb von ca. 30 °C.
Ein weiteres Herstellungsverfahren für die erfindungsgemäßen Mittel ist das Vermahlen aller Komponenten in einer Kugelmühle, z.B. einer Zentrifugalkugelmühle, einer Ringwalzenmühle oder insbesondere einer Spindelmühle.
Schließlich ist es möglich, die pulverförmigen Mittel durch Mischen aller Komponenten und die anschließende Behandlung, bevorzugt je nach verwendetem Lösemittel bei erhöhten Temperaturen, im Wirbelbett herzustellen.
Die folgenden Beispiele sollen den Gegenstand der Erfindung näher erläutern.

### 1. Blondierpulver

Es wurde die folgende Grundlage für Blondiermittel hergestellt:

| | |
|---|---|
| Pyrogene, hochdisperse Kieselsäure Typ N 20 P | 2,0 Gew.-% |
| Natriumsilikat, wasserfrei | 8,0 Gew.-% |
| Kaliumpersulfat | 35,0 Gew.-% |
| Carboxymethylcellulose, Natrium-Salz | 3,0 Gew.-% |
| Disodium EDTA | 2,0 Gew.-% |
| Ammoniumpersulfat | 12,0 Gew.-% |
| Ariabel Blue¹ | 0,4 Gew.-% |
| Magnesiumcarbonat, schwer | ad 88,0 Gew.-% |

| | |
|---|---|
| ¹ Polysulfid-Natrium-Silicoaluminat (INCI-Bezeichnung: C.I. 77007) (Goldmann) | |

Anschließend wurde die Blondiermittelgrundlage mit jeweils 12,0 Gew.-% der folgenden Lösemittel entstaubt.

| | |
|---|---|
| Rezeptur 1 | Cyclohexanon |
| Rezeptur 2 | Propylencarbonat |

Die Lösemittel wurden portionsweise zur Blondiermittelgrundlage gegeben, wobei der Ansatz zwischendurch jeweils geschüttelt wurde. Danach wurde die Rezeptur in einer Zentrifugalkugelmühle vermahlen. Alle Blondierpulver waren absolut staubfrei.
* Referenzbeispiel, außerhalb des Schutzbereichs der Ansprüche

### 1. Pulverhaarfarben*

| | |
|---|---|
| Carboxymethylcellulose, Natrium-Salz | 22,0 Gew.-% |
| Weinsäure | 10,0 Gew.-% |
| Duponol® C² | 0,5 Gew.-% |
| Natriumperborat, Monohydrat | 40,0 Gew.-% |
| Natriumsulfat | 8,5 Gew.-% |
| 1,4-Diaminobenzol, Dihydrochlorid | 7,0 Gew.-% |
| 2-Aminophenol | 1,0 Gew.-% |
| 3-Aminophenol | 1,0 Gew.-% |
| Benzylalkohol | 10,0 Gew.-% |

| | |
|---|---|
| ² Natriumlaurylsulfat, pulverförmig (INCI-Bezeichnung: Sodium Lauryl Sulfate) (DuPont) | |

Die Pulverhaarfarbe war absolut staubfrei und wurde mit Wasser im Verhältnis 1:6 gut gemischt und auf eine graue Haarsträhne aufgetragen. Nach einer Einwirkzeit von 30min bei Raumtemperatur wurde die Strähne sorgfältig ausgespült, nachshampooniert und getrocknet.
Die Strähne zeigte eine schwarze Färbung.

## Patentansprüche

1. Verwendung von 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
zum Entstauben von Blondierpulvern auf Basis mindestens einer festen Peroxoverbindung sowie mindestens eines festen Alkaliträgers und pulverförmigen Farbstoffmischungen auf Basis von mindestens einem Oxidationsfarbstoffvorprodukt enthaltend übliche kosmetische Bestandteile.

2. Pulver- oder pastenförmiges Mittel zum Blondieren menschlicher Haare auf Basis mindestens einer festen Peroxoverbiridung sowie mindestens eines festen Alkaliträgers, **dadurch gekennzeichnet, dass** es zur Entstaubung 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
enthält.

3. Mittel nach Anspruch 2, **dadurch gekennzeichnet, dass** es mindestens eine anorganische feste Peroxoverbindung enthält.

4. Pulverförmiges Mittel zum Färben menschlicher Haare, enthaltend mindestens ein Oxidationsfarbstoffvorprodukt, **dadurch gekennzeichnet, dass** es zur Entstaubung 10 bis 30 Gew.-%, bezogen auf das gesamte Mittel, mindestens eines bei 25°C flüssigen Lösemittels, ausgewählt aus der Gruppe
- der Ester Propylencarbonat, Isopropylacetat, Hexylacetat sowie Ethylglykolacetat sowie
- der Ketone Cyclohexanon, Methylisobutylketon sowie Methylheptylketon,
enthält.

5. Mittel nach Anspruch 4, **dadurch gekennzeichnet, dass** es zusätzlich mindestens einen direktziehenden Farbstoff enthält.

6. Mittel nach einem der Ansprüche 2 bis 5, **dadurch gekennzeichnet, dass** es als Lösemittel eine Verbindung, ausgewählt aus der Gruppe gebildet von Cyclohexanon sowie Propylencarbonat enthält.

7. Mittel nach einem der Ansprüche 2 bis 6, **dadurch gekennzeichnet, dass** es eine nichtionogene grenzflächenaktive Substanz in Mengen von 0,5 - 10 Gew.-% enthält.

8. Mittel nach Anspruch 7, **dadurch gekennzeichnet, dass** es eine nichtionogene grenzflächenaktive Substanz, ausgewählt aus
- alkoxylierten Fettalkoholen und Fettsäuren mit 8 bis 22 Kohlenstoffatomen und 1 bis 30 Ethylenoxid- und/oder Propylenoxid-Einheiten, die eine Alkylendgruppe, insbesondere eine Methylgruppe, am Ende der Alkoxygruppenkette aufweisen können,
- alkoxylierten Mono-, Di- und Triglyceriden,
- Polyglycerinestern und alkoxylierten Polyglycerinestern,
- Sorbitan-Fettsäureestern und alkoxylierten Sorbitan-Festtsäureestern und
- Alkylphenolen und Alkylphenolalkoxylaten mit 6 bis 21 Kohlenstoffatomen in der Alkylkette und 0 bis 30 Ethylenoxid- und/oder Propylenoxid-Einheiten,
enthält.

9. Mittel nach einem der Ansprüche 2 bis 8, **dadurch gekennzeichnet, dass** es bei 25°C feste Seifen, insbesondere Natriumstearat, in Mengen von 5-20 Gew.-%, insbesondere 10-15 Gew.-%, bezogen auf das gesamte Mittel, enthält.

10. Mittel nach einem der Ansprüche 2 bis 9, **dadurch gekennzeichnet, dass** es im übrigen frei von Ölen und flüssigen Wachsen ist, wobei Parfümöle ausgenommen sind.

## Claims

1. The use of 10 to 30% by weight, based on the preparation as a whole, of at least one solvent liquid at 25°C selected from the group of
- the esters propylene carbonate, isopropyl acetate, hexyl acetate and ethyl glycol acetate and
- the ketones cyclohexanone, methyl isobutyl ketone and methyl heptyl ketone
for de-dusting blonding powders based on at least one solid peroxo compound and at least one solid alkali source and powder-form dye mixtures based on at least one oxidation dye precursor containing typical cosmetic ingredients.

2. A powder-form or paste-form preparation for blonding human hair based on at least one solid peroxo compound and at least one solid alkali source, **characterized in that** it contains 10 to 30% by weight, based on the preparation as a whole, at least one solvent liquid at 25°C selected from the group of
- the esters propylene carbonate, isopropyl acetate, hexyl acetate and ethyl glycol acetate and
- the ketones cyclohexanone, methyl isobutyl ketone and methyl heptyl ketone
for de-dusting.

3. A preparation as claimed in claim 2, **characterized in that** it contains at least one inorganic solid peroxo compound.

4. A powder-form preparation for colouring human hair containing at least one oxidation dye precursor, **characterized in that**, for de-dusting, it contains 10 to 30% by weight, based on the preparation as a whole, of at least one solvent liquid at 25°C selected from the group of
- the esters propylene carbonate, isopropyl acetate, hexyl acetate and ethyl glycol acetate and
- the ketones cyclohexanone, methyl isobutyl ketone and methyl heptyl ketone.

5. A preparation as claimed in claim 4, **characterized in that** it additionally contains at least one substantive dye.

6. A preparation as claimed in any of claims 2 to 5, **characterized in that** it contains a compound selected from the group consisting of cyclohexanone and propylene carbonate as solvent.

7. A preparation as claimed in any of claims 2 to 6, **characterized in that** it contains a nonionic surfactant in quantities of 0.5 to 10% by weight.

8. A preparation as claimed in claim 7, **characterized in that** it contains a nonionic surfactant selected from
- alkoxylated fatty alcohols and fatty acids containing 8 to 22 carbon atoms and 1 to 30 ethylene oxide and/or propylene oxide units which may carry a terminal alkyl group, more particularly a methyl group, at the end of the alkoxy group chain,
- alkoxylated mono-, di- and triglycerides,
- polyglycerol esters and alkoxylated polyglycerol esters,
- sorbitan fatty acid esters and alkoxylated sorbitan fatty acid esters and
- alkyl phenols and alkyl phenol alkoxylates containing 6 to 21 carbon atoms in the alkyl chain and 0 to 30 ethylene oxide and/or propylene oxide units.

9. A preparation as claimed in any of claims 2 to 8, **characterized in that** it contains soaps solid at 25°C, more particularly sodium stearate, in quantities of 5 to 20% by weight and more particularly in quantities of 10 to 15% by weight, based on the preparation as a whole.

10. A preparation as claimed in any of claims 2 to 9, **characterized in that** it is free from oils and liquid waxes except perfume oils.

## Revendications

1. Utilisation de 10 à 30% en poids, par rapport à l'agent total, d'au moins un solvant liquide à 25°C, choisi dans le groupe
- des esters carbonate de propylène, acétate d'isopropyle, acétate d'hexyle ainsi qu'acétate d'éthylglycol ainsi que
- des cétones cyclohexanone, méthylisobutylcétone ainsi que méthylheptylcétone,
pour dépoussiérer des poudres de décoloration à base d'au moins un composé peroxo solide ainsi que d'au moins un support alcalin solide et des mélanges de colorants sous forme de poudre à base d'au moins un précurseur de colorant d'oxydation contenant des constituants cosmétiques usuels.

2. Agent sous forme de poudre ou de pâte pour décolorer des cheveux humains à base d'au moins un composé peroxo solide ainsi que d'au moins un support alcalin solide, **caractérisé en ce qu'**il contient, pour le dépoussiérage, 10 à 30% en poids, par rapport à l'agent total, d'au moins un solvant liquide à 25°C, choisi dans le groupe
- des esters carbonate de propylène, acétate d'isopropyle, acétate d'hexyle ainsi qu'acétate d'éthylglycol ainsi que
- des cétones cyclohexanone, méthylisobutylcétone ainsi que méthylheptylcétone.

3. Agent selon la revendication 2, **caractérisé en ce qu'**il contient au moins un composé peroxo solide inorganique.

4. Agent sous forme de poudre pour teindre des cheveux humains, contenant au moins un précurseur de colorant d'oxydation, **caractérisé en ce qu'**il contient, pour le dépoussiérage, 10 à 30% en poids, par rapport à l'agent total, d'au moins un solvant liquide à 25°C, choisi dans le groupe
- des esters carbonate de propylène, acétate d'isopropyle, acétate d'hexyle ainsi qu'acétate d'éthylglycol ainsi que
- des cétones cyclohexanone, méthylisobutylcétone ainsi que méthylheptylcétone.

5. Agent selon la revendication 4, **caractérisé en ce qu'**il contient en outre au moins un colorant montant directement sur la fibre.

6. Agent selon l'une quelconque des revendications 2 à 5, **caractérisé en ce qu'**il contient comme solvant un composé choisi dans le groupe formé par la cyclohexanone et le carbonate de propylène.

7. Agent selon l'une quelconque des revendications 2 à 6, **caractérisé en ce qu'**il contient une substance tensioactive non ionogène en des quantités de 0,5 à 10% en poids.

8. Agent selon la revendication 7, **caractérisé en ce qu'**il contient une substance tensioactive non ionogène choisie parmi
- les alcools et les acides gras alcoxylés comprenant 8 à 22 atomes de carbone et 1 à 30 unités d'oxyde d'éthylène et/ou d'oxyde de propylène, qui peuvent présenter un groupe terminal alkyle, en particulier un groupe méthyle, en l'extrémité de la chaîne de groupes alcoxy,
- les monoglycérides, diglycérides et triglycérides alcoxylés,
- les esters de polyglycérol et les esters alcoxylés de polyglycérol,
- les esters d'acide gras de sorbitane et les esters alcoxylés d'acide gras de sorbitane et
- les alkylphénols et les alcoxylates d'alkylphénol comprenant 6 à 21 atomes de carbone dans la chaîne alkyle et 0 à 30 unités d'oxyde d'éthylène et/ou d'oxyde de propylène.

9. Agent selon l'une quelconque des revendications 2 à 8, **caractérisé en ce qu'**il contient des savons solides à 25°C, en particulier le stéarate de sodium, en des quantités de 5 à 20% en poids, en particulier de 10 à 15% en poids, par rapport à l'agent total.

10. Agent selon l'une quelconque des revendications 2 à 9, **caractérisé en ce qu'**il est pour le reste exempt d'huiles et de cires liquides, à l'exception d'huiles parfumées.
